# EUROPÄISCHE PATENTSCHRIFT

(11) **EP 2 072 499 B1**
(45) Veröffentlichungstag und Bekanntmachung des Hinweises auf die Patenterteilung: **22.02.2012**
(21) Anmeldenummer: 08169741.9
(22) Anmeldetag: 24.11.2008
(51) Int. Cl.: C07C 319/14, C07C 323/52

(54) **Verfahren zur Herstellung von Thiodiglycolsäuredialkylester**
Method for manufacturing thiodiglycolic dialkyl esters
Procédé de fabrication de l'éster dialkyle de l'acide thiodiglycolique

(30) Priorität: 21.12.2007 DE 102007062282
(43) Veröffentlichungstag der Anmeldung: 24.06.2009
(73) Patentinhaber: Saltigo GmbH, 40764 Langenfeld (DE)
(72) Erfinder: Rodefeld, Lars, 51375 Leverkusen (DE); Broda, Witold, 53819 Neunkirchen-Seelscheid (DE); Sommer, Joachim, 61200 Wölfersheim (DE); Westen, Joachim, 42659 Solingen (DE); Richter, Hartmut, 40764 Langenfeld (DE)
(74) Vertreter: Pettrich, Klaus-Günter

(56) Entgegenhaltungen:
- EP-A- 0 697 401
- WO-A-00/45451
- US-A- 2 262 686
- DATABASE WPI Week 199105 Thomson Scientific, London, GB; AN 1991-033737 XP002522073 & JP 02 304061 A (BARAIT KOGYO KK) 17. Dezember 1990 (1990-12-17)

## Beschreibung

Die Erfindung betrifft ein neues Verfahren zur Herstellung von C₁-C₁₀-Thiodiglycolsäuredialkylester.

Thiodiglycolsäuredialkylester sind wichtige Vorprodukte für die Herstellung von Spezialchemikalien zum Beispiel für den Einsatz in elektronisch leitfähigen Polymeren.

Die Synthese von Thiodiglycolsäurediester ist prinzipiell bereits seit langem bekannt.

Es gibt im wesentlichen zwei Wege Thiodiglycolsäuredialkylester zu synthetisieren:
a) Veresterung von Thiodiglycolsäure mit Alkoholen unter saurer Katalyse.
b) Umsetzung von Chloressigsäureester mit Natriumsulfid.

Ein wesentlicher Nachteil der Variante a) ist, dass für die Veresterung der Thiodiglycolsäure mit Alkoholen mit Hilfe von Salzsäure (Schulze, Zeitschrift für Chemie 1865, S. 78) oder mit Hilfe von Schwefelsäure (Seka, Berichte 58, 1925, S. 1786) kristalline Thiodiglycolsäure benötigt wird. Dies bedeutet, dass die sehr gut wasserlösliche Thiodiglycolsäure aus der Wasserphase separiert werden muss. Dabei verbleibt stets ein Teil der Thiodiglycolsäure in der Mutterlauge und außerdem fällt bei der Isolierung als Feststoff die Thiodiglycolsäure zusammen mit anorganischen Salzen an, so dass sie nocheinmal umkristallisiert werden muss. Eine Veresterung aus der wässrigen Lösung liefert daher unbefriedigende Resultate (US 2,425,225).

Um die aufwändige Isolierung der Thiodiglycolsäure als Feststoff zu umgehen, wird in der US 2,425,225 ein Verfahren beschrieben, bei dem die Thiodiglycolsäure direkt mit dem zu veresterenden Alkohol aus der wässrigen Phase extrahiert wird. Allerdings funktioniert dieses Verfahren nur mit Alkoholen mit mehr als drei Kohlenstoffatomen und ist daher ungeeignet für die Synthese von Thiodiglycolsäure-methylester, -ethylester und -propylester, da die entsprechenden Alkohole mit weniger als vier Kohlenstoffatomen vollständig in Wasser löslich sind und daher nicht als Extraktionsmittel fungieren können.

Eine besondere Schwierigkeit bei der Umsetzung von Chloressigsäureester mit Natriumsulfid liegt darin, dass Natriumsulfid eine sehr starke Base ist, während Chloressigsäureester sehr pHempfindlich sind und leicht verseifen.

Leitet man eine wässrige Natriumsulfidlösung in Chloressigsäuremethylester ein, erhält man aufgrund der Verseifung des Edukts und/oder des Produkts nur mäßige Ausbeuten an Thiodiglycolsäuremethylester. In der WO 00/45451 wird bei der Umsetzung von Bromessigsäureethylester mit Natriumsulfid eine Ausbeute von 39% angegeben.

Eine Möglichkeit die Verseifung des Chloressigsäureesters zu vermeiden, ist, wie in der US 2,262,686 beschrieben, die Reaktion in einem inerten Lösungsmittel wie Aceton durchzuführen. Bei dieser wasserfreien Variante zur Synthese von Thiodiglycolsäureester wird wasserfreies Natriumsulfid benötigt, welches deutlich teurer als wasserhaltiges ist, und die Reaktionszeit ist mit 15 bis 20 h Kochen am Rückfluss unverhältnismäßig lang.

Aufgabe der vorliegenden Erfindung war es, ein Verfahren zur Herstellung eines Thiodiglycolsäuredialkylesters zur Verfügung zu stellen, welches das gewünschte Produkt auf vereinfachte Weise und mit besserer Ausbeute erbringt, als nach dem bisherigen Stand der Technik.

Erstaunlicherweise konnte festgestellt werden, dass durch Einstellen geeigneter Reaktionsbedingungen die Umsetzung von Chloressigsäuremethylester mit Alkalisulfid oder Alkalihydrogensulfid zum Thiodiglycolsäuredimethylester in wässriger Lösung doch sehr hohe Ausbeuten liefert. Entscheidend ist, dass die Sulfidverbindung schnell abreagiert und damit der pH-Wert in der Lösung im Bereich 5 < pH < 8 gehalten wird.

Gegenstand der Erfindung ist daher ein Verfahren zur Herstellung von Thiodiglycolsäurealkylester der allgemeinen Formel (I)

R-OOC-CH2-S-CH2-COO-R (I),

wobei R für einen Rest von verzweigten oder unverzweigten C₁ bis C₁₀-Alkyl steht,
dadurch gekennzeichnet, dass ein Halogenessigsäurealkylester der allgemeinen Formel (II)

X-CH2-COO-R (II),

wobei X für ein Chlor- oder Bromatom steht und R die Bedeutung wie für Verbindungen der Formel (I) angegeben hat,
mit einer wässrigen Lösung von Alkalisulfid oder Alkalihydrogensulfid in Gegenwart einer wässrigen pH-Puffer-Lösung im pH-Bereich zwischen 5 und 8 umgesetzt wird.

Bevorzugt ist die wässrige Pufferlösung eine Dialkalihydrogenphosphat- oder Alkalidihydrogenphosphat- oder Ammoniumacetat- oder Ammoniumchlorid-Pufferlösung. Beispiele für Dialkalihydrogenphosphat sind K₂HPO₄ oder Na₂HPO₄, Beispiele für Alkalidihydrogenphosphat sind KH₂PO₄ und NaH₂PO₄.

Als Sulfidquelle können wässrige Lösungen sowohl von Alkalisulfid (Natrium- oder Kaliumsulfid) als auch von Alkalihydrogensulfid (Natrium- oder Kaliumhydrogensulfid) eingesetzt werden. Die Konzentration der wässrigen Alkalisulfidlösung liegt dabei zwischen 5 und 20 Gew% und die der Alkalihydrogensulfidlösung liegt zwischen 5 und 50 Gew%.

Die Zudosierung der Reagenzien zur wässrigen Pufferlösung kann unter Rühren auf an sich bekannte Weise, z.B. in einem beheizbaren Doppelmantelgefäß über Pumpen, durch getrennte Leitungen oder über einen statischen Mischer, erfolgen.

Üblicherweise wird der C₁-C₁₀-Halogenessigsäurealkylester zusammen mit dem Alkali- bzw. Alkalihydrogensulfid in einem Molverhältnis zwischen 1:1 bis 3:1 zudosiert. Bevorzugt beträgt dieses Molverhältnis 2:1. Die Zudosierung kann dabei simultan, oder portionsweise, bevorzugt aber simultan erfolgen.

Die simultane Dosierung erfolgt dabei in der Regel über einen Zeitraum von 0,5 bis 24 Stunden. Die Temperatur, bei der die Dosierung erfolgt, sollte im Bereich zwischen 10 und 60°C, bevorzugt 20 bis 40°C.

Vorzugsweise wird die Reaktion in Gegenwart eines Phasentransferkatalysators durchgeführt. Bevorzugt sind dabei die kommerziell erhältlichen Katalysatoren Tetrabutylammoniumchlorid, Tributylmethylammoniumchlorid, Methyltrioctylammoniumchlorid, Methyltridecylammoniumchlorid, Polyethylenglycol 400 - 40.000, Kronenether, Tris[2-(2-methoxyethoxy)ethyl]amine oder Trialkylphosphoniumsalze. Besonders bevorzugt wird als Phasentransferkatalysator Tetrabutylammoniumchlorid oder Polyethylenglycol 400 eingesetzt.

Es muss darauf geachtet werden, dass der pH-Wert während der Reaktion zwischen 5 und 8 gehalten wird.

Nach erfolgter Reaktion wird das Rohprodukt aus der wässrigen Pufferlösung abgetrennt. Dies kann z.B. durch Extraktion erfolgen. Hierzu wird die Reaktionslösung mit einem mit Wasser nicht mischbaren Lösungsmittel versetzt und die wässrige Phase von der organischen Phase abgetrennt. Aus der organischen Phase kann dann anschließend, z.B. durch Destillation, das Extraktionsmittel abgetrennt werden. Der Rückstand enthält das gewünschte Produkt in Ausbeuten von 88 bis 95 % der Theorie.

Als Extraktionsmittel zur Abtrennung des Rohprodukts aus der wässrigen Pufferlösung können verzweigte oder unverzweigte C₂-C₄-Dialkylether, wie Diethylether (DEE) und Methyl-tert.-butylether (MTBE), oder verzweigte oder unverzweigte Dialkyl-Ketone, wie Methylisobutylketon (MIBK), oder verzweigte oder unverzweigte C₄-C₁₀-Kohlenwasserstoffe wie Pentan, Hexan, Heptan oder Cyclohexan, oder aromatische Verbindungen wie Benzol, Toluol, Xylol oder Dichlorbenzol eingesetzt werden. Bevorzugt wird Toluol als Extraktionsmittel eingesetzt.

Nach dem erfindungsgemäßen Verfahren können Thiodiglycolsäuredialkylester mit C₁-C₁₀-Alkylresten in den Esterteilen durch den Einsatz des entsprechenden C₁-C₁₀-Halogcnessigsäurealkylesters hergestellt werden. Bevorzugt stellt man die C₁-C₄-Alkylester, also Thiodiglycolsäuredimethyl-, Thiodiglycolsäurediethyl-, Thiodiglycolsäuredipropyl- oder Thiodiglycolsäuredibutylester her. Besonders bevorzugt sind dabei Thiodiglycolsäuredimethyl- und Thiosäurediglycolsäurediethylester.

Die nach beschriebenen Verfahren gewonnenen Thiodiglycolsäuredialkylester können ohne destillative Aufreinigung direkt weiterverarbeitet werden. Aufgrund der nahezu quantitativen und schnellen Umsetzung von Natriumsulfid enthalten weder die Abluft noch das Abwasser nennenswerte Mengen an Schwefelwasserstoff.

Die folgenden Beispiele sollen die Erfindung weiter erläutern.

### Beispiel 1

In einem 1 Itr.-Doppelmantelglasreaktor wurden 17,9 g Natriumdihydrogenphosphat-Dihydrat in 84,2 g Wasser gelöst (pH = 3,9) und mit 6,7 g Natronlauge (32%ig) auf pH = 6,0 gestellt. Die Pufferlösung wurde auf 33°C erwärmt und mit 13,0 g Tributylmethylammoniumchloridlösung (75%ig in Wasser) und 40,0 g Chloressigsäuremethylester versetzt. Simultan wurden 611,2 g Natriumsulfidlösung (16%ig in Wasser) sowie 182,1 g Chloressigsäuremethylester innerhalb von 2 h bei 30 - 35°C zudosiert. Anschließend wurden noch weitere 19,3 g Natriumsulfidlösung (16%ig in Wasser) zudosiert und 1 h bei 33°C nachgerührt. Die Reaktionslösung wurde mit 130 ml Toluol versetzt, kräftig gerührt und anschließend wurde die untere Phase abgetrennt. Nach Abdestillieren des Toluols im Vakuum bei ca. 300 mbar wurden 226,6 g einer klaren Flüssigkeit mit 93% Thiodiglycolsäuredimethylester und 6% Toluol erhalten. Dies entspricht einer Ausbeute der Theorie von 93%.

### Beispiel 2

In einem 1 ltr.-Doppelmantelglasreaktor wurden 35,8 g Natriumdihydrogenphosphat-Dihydrat in 168,4 g Wasser gelöst (pH = 3,9) und mit 24,4 g Natronlauge (32%ig) auf pH = 7,0 gestellt. Die Pufferlösung wurde auf 33°C erwärmt und mit 13,0 g Tributylmethylammoniumchloridlösung (75%ig in Wasser) und 40,0 g Chloressigsäuremethylester versetzt. Simultan wurden 269,8 g Natriumhydrogensulfidlösung (26%ig in Wasser) sowie 182,1 g Chloressigsäuremethylester innerhalb von 2 h bei 30 - 35°C zudosiert. Dabei wurde durch simultane Zugabe von Natronlauge (32%ig in Wasser) der pH = 7 gehalten. Anschließend wurden noch weitere 8,5 g Natriumhydrogensulfidlösung (26% in Wasser) zudosiert und 1 h bei 33°C nachgerührt. Die Reaktionslösung wurde mit 130 ml Toluol versetzt und kräftig gerührt. Anschließend wurde die untere Phase abgetrennt. Nach Abdestillieren des Toluols im Vakuum bei ca. 300 mbar wurden 236,4 g einer klaren Flüssigkeit mit 91% Thiodiglycolsäuredimethylester und 6% Toluol erhalten. Dies entspricht einer Ausbeute der Theorie von 95%.

### Beispiel 3

In einem 1 ltr.-Doppelmantelglasreaktor wurden 17,9 g Natriumdihydrogenphosphat-Dihydrat in 84,2 g Wasser gelöst (pH = 3,9) und mit 6,7 g Natronlauge (32%ig) auf pH = 6,0 gestellt. Die Pufferlösung wurde auf 33°C erwärmt und mit 13,0 g Tributylmethylammoniumchloridlösung (75%ig in Wasser) und 40,0 g Chloressigsäuremethylester versetzt. Simultan wurden 611,2 g Natriumsulfidlösung (16%ig in Wasser) sowie 182,1 g Chloressigsäuremethylester innerhalb von 2 h bei 30 - 35°C zudosiert. Anschließend wurden noch weitere 19,3 g Natriumsulfidlösung (16%ig in Wasser) zudosiert und 1 h bei 33°C nachgerührt. Die Reaktionslösung wurde mit 130 ml Toluol versetzt und kräftig gerührt. Anschließend wurde die untere Phase abgetrennt. Nach Abdestillieren des Toluols im Vakuum bei ca. 300 mbar wurden 226,6 g einer klaren Flüssigkeit mit 93% Thiodiglycolsäuredimethylester und 6% Toluol erhalten. Dies entspricht einer Ausbeute der Theorie von 93%.

### Beispiel 4

In einem 1 Itr.-Doppelmantelglasreaktor wurden 35,8 g Natriumdihydrogenphosphat-Dihydrat in 168,4 g Wasser gelöst (pH = 3,9) und mit 11,4 g Natronlauge (32%ig) auf pH = 6,0 gestellt. Die Pufferlösung wurde auf 33°C erwärmt und mit 13,0 g Polyethylenglycol 400 und 40,0 g Chloressigsäuremethylester versetzt. Simultan wurden 611,2 g Natriumsulfidlösung (16%ig in Wasser) sowie 182,1 g Chloressigsäuremethylester innerhalb von 2 h bei 30 - 35°C zudosiert. Anschließend wurden noch weitere 19,3 g Natriumsulfidlösung (16%ig in Wasser) zudosiert und 2 h bei 33°C nachgerührt. Die Reaktionslösung wurde mit 130 ml Toluol versetzt und kräftig gerührt. Anschließend wurde die untere Phase abgetrennt. Nach Abdestillieren des Toluols im Vakuum bei ca. 300 mbar wurden 227,2 g einer klaren Flüssigkeit mit 88% Thiodiglycolsäuredimethylester, 1% Methylthioglycolat, 1% Chloressigsäurethylester und 6% Toluol erhalten. Dies entspricht einer Ausbeute der Theorie von 90%.

### Beispiel 5

In einem 1 1tr.-Doppelmantelglasreaktor wurden 15,8 g Eisessig in 168,4 g Wasser gelöst (pH = 2,3) und mit 19,0 g wässriger Ammoniaklösung (26%ig) auf pH = 6,0 gestellt. Die Pufferlösung wurde auf 33°C erwärmt und mit 16,0 g Tributylmethylammoniumchloridlösung (75%ig in Wasser) und 40,0 g Chloressigsäuremethylester versetzt. Simultan wurden 611,2 g Natriumsulfidlösung (16%ig in Wasser) sowie 182,1 g Chloressigsäuremethylester innerhalb von 2 h bei 30 - 35°C zudosiert. Anschließend wurden noch weitere 19,3 g Natriumsulfidlösung (16%ig in Wasser) zudosiert und 1 h bei 33°C nachgerührt. Die Reaktionslösung wurde mit 130 ml Toluol versetzt und kräftig gerührt. Anschließend wurde die untere Phase abgetrennt. Nach Abdestillieren des Toluols im Vakuum bei ca. 300 mbar wurden 223,8 g einer klaren Flüssigkeit mit 92% Thiodiglycolsäuredimethylester und 6% Toluol erhalten. Dies entspricht einer Ausbeute der Theorie von 90%.

### Beispiel 6

In einem 1 ltr.-Doppelmantelglasreaktor wurden 35,8 g Natriumdihydrogenphosphat-Dihydrat in 168,4 g Wasser gelöst (pH = 3,9) und mit 12,2 g Natronlauge (32%ig) auf pH = 6,0 gestellt. Die Pufferlösung wurde auf 33°C erwärmt und mit 13,0 g Tributylmethylammoniumchloridlösung (75%ig in Wasser) und 40,0 g Chloressigsäureethylester versetzt. Simultan wurden 611,2 g Natriumsulfidlösung (16%ig in Wasser) sowie 275,3 g Chloressigsäureethylester innerhalb von 2 h bei 30 - 35°C zudosiert. Anschließend wurden noch weitere 19,3 g Natriumsulfidlösung (16%ig in Wasser) zudosiert und 1 h bei 33°C nachgerührt. Anschließend wurden noch weitere 19,3 g Natriumsulfidlösung (16%ig in Wasser) zudosiert und 2 h bei 33°C nachgerührt. Die Reaktionslösung wurde mit 130 ml Toluol versetzt und kräftig gerührt. Anschließend wurde die untere Phase abgetrennt. Nach Abdestillieren des Toluols im Vakuum bei ca. 300 mbar wurden 277,2 g einer klaren Flüssigkeit mit 90% Thiodiglycolsäurediethylester und 9% Toluol erhalten. Dies entspricht einer Ausbeute der Theorie von 95%.

## Patentansprüche

1. Verfahren zur Herstellung von Thiodiglycolsäurealkylester der allgemeinen Formel (I)
R-OOC-CH2-S-CH2-COO-R (I),
wobei R für einen Rest von verzweigten oder unverzweigten C₁ bis C₁₀-Alkyl steht, **dadurch gekennzeichnet, dass** ein Halogenessigsäurealkylester der allgemeinen Formel (II)
X-CH₂-COO-R (II),
wobei X für ein Chlor- oder Bromatom steht und R die Bedeutung wie für Verbindungen der Formel (I) angegeben hat,
mit einer wässrigen Lösung von Alkalisulfid oder Alkalihydrogensulfid in Gegenwart einer wässrigen pH-Puffer-Lösung im pH-Bereich zwischen 5 und 8 umgesetzt wird.

2. Verfahren nach Anspruch 1, **dadurch gekennzeichnet, dass** das Verfahren in Gegenwart eines Phasentransferkatalysators durchgeführt wird.

3. Verfahren nach einander Ansprüche 1 bis 2, **dadurch gekennzeichnet, dass** die wässrige Pufferlösung einen Dialkalihydrogenphosphat-, einen Alkalidihydrogenphosphat-, einen Natriumhydrogencarbonat-, einen Ammoniumacetat- oder einen Ammoniumchlorid-Puffer enthält.

4. Verfahren nach einem der Ansprüche 1 bis 3, **dadurch gekennzeichnet, dass** der pH-Bereich zwischen 6 und 8 liegt.

5. Verfahren nach einem der Ansprüche 1 bis 4, **dadurch gekennzeichnet, dass** als wässrige Alkalisulfid-Lösung eine wässrige Natriumsulfid-Lösung mit einem Gehalt zwischen 5 und 30 Gew% oder eine wässrige Natriumhydrogensulfid-Lösung mit einem Gehalt zwischen 5 und 50 Gew% eingesetzt wird.

6. Verfahren nach einem der Ansprüche 1 bis 5, **dadurch gekennzeichnet, dass** der Halogenessigsäurealkylester der Formel (II) ein Chloressigsäure-methylester, -ethylester, -propylester, -butylester, -pentylester, -hexylester oder -cyclohexylester oder ein Bromessigsäure-methylester, -ethylester, -propylester, -butylester -pentylester, -hexylester oder -cyclohexylester ist.

7. Verfahren nach einem der Ansprüche 1 bis 6, **dadurch gekennzeichnet, dass** der Halogenessigsäurealkylester der Formel (II) und die Alkalisufid- oder Alkalihydrogensulfid-Lösung in einem Molverhältnis von 1:1 bis 3:1 zueinander simultan zudosiert werden.

8. Verfahren nach einem der Ansprüche 1 bis 7, **dadurch gekennzeichnet, dass** die Temperatur der Reaktionslösung im Bereich von 0 bis 60°C liegt.

9. Verfahren nach einem der Ansprüche 2 bis 8, **dadurch gekennzeichnet, dass** als Phasentransferkatalysator Tetrabutylammoniumchlorid, Tributylmethylammoniumchlorid, Methyltrioctylammoniumchlorid, Methyltridecylammoniumchlorid, Polyethylenglycol 400 - 40.000, Kronenether, Tris[2-(2-methoxyethoxy)ethyl]amine oder ein Trialkylphosphoniumsalz eingesetzt wird.

10. Verfahren nach einem der Ansprüche 1 bis 9, **dadurch gekennzeichnet, dass** man den C₁-C₁₀-Thiodiglycolsäuredialkylester durch Extraktion mit einem mit Wasser nicht mischbarem organischen Lösungsmittel von der wässrigen Reaktionslösung abtrennt.

## Claims

1. Process for preparing alkyl thiodiglycolates of the general formula (I)
R-OOC-CH2-S-CH2-COO-R (I)
where R is a of branched or unbranched C₁ to C₁₀-alkyl radical,
**characterized in that** an alkyl haloacetate of the general formula (II)
X-CH2-COO-R (II)
where X is a chlorine or bromine atom and R is as defined for compounds of the formula (I)
is reacted with an aqueous solution of alkali metal sulphide or alkali metal hydrogensulphide in the presence of an aqueous pH buffer solution in the pH range between 5 and 8.

2. Process according to Claim 1, **characterized in that** the process is performed in the presence of a phase transfer catalyst.

3. Process according to either of Claims 1 and 2, **characterized in that** the aqueous buffer solution comprises a dialkali metal hydrogenphosphate buffer, an alkali metal dihydrogenphosphate buffer, a sodium hydrogencarbonate buffer, an ammonium acetate buffer or an ammonium chloride buffer.

4. Process according to any of Claims 1 to 3, **characterized in that** the pH range is between 6 and 8.

5. Process according to any of Claims 1 to 4, **characterized in that** the aqueous alkali metal sulphide solution used is an aqueous sodium sulphide solution having a content between 5 and 30% by weight or an aqueous sodium hydrogensulphide solution having a content between 5 and 50% by weight.

6. Process according to any of Claims 1 to 5, **characterized in that** the alkyl haloacetate of the formula (II) is a methyl, ethyl, propyl, butyl, pentyl, hexyl or cyclohexyl chloroacetate or a methyl, ethyl, propyl, butyl, pentyl, hexyl or cyclohexyl bromoacetate.

7. Process according to any of Claims 1 to 6, **characterized in that** the alkyl haloacetate of the formula (II) and the alkali metal sulphide or alkali metal hydrogensulphide solution are metered in simultaneously in a molar ratio of 1:1 to 3:1 relative to one another.

8. Process according to any of Claims 1 to 7, **characterized in that** the temperature of the reaction solution is in the range of 0 to 60°C.

9. Process according to any of Claims 2 to 8, **characterized in that** the phase transfer catalyst used is tetrabutylammonium chloride, tributyl-methylammonium chloride, methyltrioctylammonium chloride, methyltridecylammonium chloride, polyethylene glycol 400 - 40 000, crown ethers, tris[2-(2-methoxyethoxy)ethyl]amine or a trialkyl-phosphonium salt.

10. Process according to any of Claims 1 to 9, **characterized in that** the dialkyl C₁-C₁₀-thiodiglycolate is removed from the aqueous reaction solution with a water-immiscible organic solvent.

## Revendications

1. Procédé pour la préparation de thiodiglycolate d'alkyle de formule générale (I).
R-OOC-CH₂-S-CH₂-COO-R (I)
R représentant un radical alkyle en C₁-C₁₀ ramifié ou non ramifié,
**caractérisé en ce qu'**on fait réagir un halogénoacétate d'alkyle de formule générale (II)
X-CH₂-COO-R (II),
X représentant un atome de chlore ou de brome et R ayant la signification indiquée pour les composés de formule (I),
avec une solution aqueuse de sulfure de métal alcalin ou d'hydrogénosulfure de métal alcalin en présence d'une solution aqueuse de tampon de pH dans l'intervalle de pH compris entre 5 et 8.

2. Procédé selon la revendication 1, **caractérisé en ce que** le procédé est effectué en présence d'un catalyseur de transfert de phase.

3. Procédé selon l'une quelconque des revendications 1 et 2, **caractérisé en ce que** la solution aqueuse de tampon contient un tampon monohydrogénophosphate de métal alcalin, un tampon dihydrogénophosphate de métal alcalin, un tampon hydrogénocarbonate de sodium, un tampon acétate d'ammonium ou un tampon chlorure d'ammonium.

4. Procédé selon l'une quelconque des revendications 1 à 3, **caractérisé en ce que** l'intervalle de pH se situe entre 6 et 8.

5. Procédé selon l'une quelconque des revendications 1 à 4, **caractérisé en ce qu'**on utilise comme solution aqueuse de sulfure de métal alcalin une solution aqueuse de sulfure de sodium ayant une teneur comprise entre 5 et 30 % en poids ou une solution aqueuse d'hydrogénosulfure de sodium ayant une teneur comprise entre 5 et 50 % en poids.

6. Procédé selon l'une quelconque des revendications 1 à 5, **caractérisé en ce que** l'halogénoacétate d'alkyle de formule (II) est un chloracétate de méthyle, éthyle, propyle, butyle, pentyle, hexyle ou cyclohexyle ou un bromoacétate de méthyle, éthyle, propyle, butyle, pentyle, hexyle ou cyclohexyle.

7. Procédé selon l'une quelconque des revendications 1 à 6, **caractérisé en ce qu'**on ajoute par addition dosée simultanément l'halogénoacétate d'alkyle de formule (II) et la solution de sulfure de métal alcalin ou d'hydrogénosulfure de métal alcalin en un rapport molaire l'un à l'autre de 1:1 à 3:1.

8. Procédé selon l'une quelconque des revendications 1 à 7, **caractérisé en ce que** la température de la solution réactionnelle se situe dans la plage allant de 0 à 60 °C.

9. Procédé selon l'une quelconque des revendications 2 à 8, **caractérisé en ce qu'**on utilise come catalyseur de transfert de phase le chlorure de tétrabutylammonium, le chlorure de tributylméthyl-ammonium, le chlorure de méthyltrioctylammonium, le chlorure de méthyltridécylammonium, un polyéthylène-glycol 400 - 40 000, des éthers-couronnes, des tris[2-(2-méthoxyéthoxy)éthyl]amines ou un sel de trialkyl-phosphonium.

10. Procédé selon l'une quelconque des revendications 1 à 9, **caractérisé en ce qu'**on sépare le thiodiglycolate de dialkyle en C₁-C₁₀ de la solution réactionnelle aqueuse par extraction avec un solvant organique non miscible à l'eau.
